# EUROPEAN PATENT APPLICATION

(11) **EP 3 608 318 A1**
(43) Date of publication of application: **12.02.2020**
(21) Application number: 18290093.6
(22) Date of filing: 09.08.2018
(51) Int. Cl.: C07D 471/08, A61K 31/46, A61P 31/04

(54) **DIAAZABICYCLOOCTANONE DERIVATIVES AS ANTIBACTERIALS**

(71) Applicant: Antabio SAS, 31670 Labège (FR)
(72) Inventor: The designation of the inventor has not yet been filed
(74) Representative: J A Kemp LLP

(57) **Abstract**

The invention relates to a compound which is a diazabicyclooctanone of Formula (I) or a pharmaceutically acceptable salt thereof: 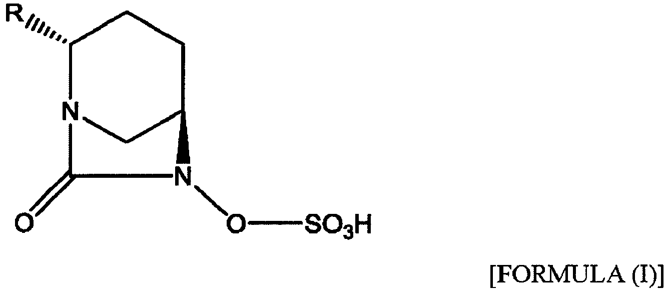 wherein R is as defined herein. The compounds are useful in the treatment of bacterial infection, in particular they are useful in reducing bacterial resistance to antibiotics. They are also useful in the treatment of bacteria which express serine-P-lactamase enzymes, in combination with antibiotics.

## Description

### Field of the Invention

The invention relates to compounds which are inhibitors of serine beta lactamase enzymes, and compositions containing the compounds. The compounds are useful in the treatment of bacterial infection. The invention also relates to combinations of the compounds with other active agents useful in the treatment of bacterial infection.

### Background

Antibiotic resistance in pathogenic bacteria is a major public health threat worldwide. Resistance of bacteria to β-lactam antibiotics caused by the hydrolysis of β-lactam antibiotics by β-lactamase enzymes is of particular concern. The enzymes affect the hydrolytic cleavage of the β-lactam ring, rendering the β-lactam antibiotic inactive. Beta-lactamases belong to two structurally and mechanistically unrelated families of enzymes: the serine β-lactamases (SBLs; classes A, C and D) which use an active serine to cleave the β-lactam in a covalent mechanism, and the metallo β-lactamases (MBLs; class B) which use metal ion catalysis to directly hydrolyze the β-lactam without the formation of a covalent intermediate.

To counter the threat of emerging resistance, in 1981 the Streptomyces clavuligerus natural product clavulanic acid (an SBL inhibitor: see Scheme 1 in the General Synthetic Methodology section below), was introduced as part of a combination together with the β-lactam antibiotic amoxicillin (as Augmentin) (see De Koning, G.A. et al, 1981, J. Antimicrobial Chemotherapy 8, 81-82). More recently, there has been renewed interest in the field of β-lactamase inhibitor discovery in order to counter the threat from newer β-lactamases which are not inhibited by clavulanic acid, such as the extended spectrum β-lactamases (ESBLs) and carbapenemases. This has led to the development of new synthetic classes of inhibitors, such as the diazabicyclooctonanes (DBOs), as exemplified by avibactam which is in clinical use in combination with ceftazidime (Mawal, Y. et al, 2015, Expert Rev. Clin. Pharmacol. 8, (6), 691-707).

The carbapenems such as meropenem and imipenem are widely regarded as the drugs of choice for the treatment of severe infections caused by ESBL-producing

Enterobacteriaceae. While avibactam is a good nanomolar inhibitor of many of the clinically-relevant SBLs that hydrolyse carbapenems, it is poor against the OXA family which are among the most prevalent carbapenemases in Europe and the Middle East (Cantón R et al, European Network on Carbapenemases, (2012), Rapid evolution and spread of carbapenemases among Enterobacteriaceae in Europe. Clin Microbiol Infect 18:413-431; and Nordmann P et al, 2011, Global spread of carbapenemase-producing Enterobacteriaceae. Emerg Infect Dis 17:1791-1798).

There is therefore a need to provide new SBL inhibitors, in particular inhibitors capable of inhibiting the OXA family of β-lactamases. There is also a need to provide broad spectrum inhibitors capable of inhibiting a range of SBLs, including the extended spectrum β-lactamases (ESBLs) and carbapenemases. The present invention aims to address some or all of these issues.

### Summary of the Invention

The present inventors have surprisingly found that novel analogues in the DBO field have unexpected activity against SBL enzymes including the OXA variants. As such, these compounds are expected to be of use as an adjunct to carbapenems in treating infectious disease.

Accordingly, the invention provides a compound as set out in aspect [1]. The compounds are useful in the treatment of bacterial infection, in particular they are useful in removing or reducing SBL-derived bacterial resistance to antibiotics. They are therefore useful in the treatment of infection caused by SBL-producing bacteria, e.g. in combination with antibiotics. Thus, the treatment or prevention of bacteria is typically carried out by administering the compound of the invention in combination with an antibiotic agent. The compounds are also useful in combination with metallo-β-lactamase (MBL) inhibitors, in particular where the bacterial infection is caused by both SBL and MBL-producing bacteria.

The present invention, in particular, provides:
1. A compound which is a diazabicyclooctanone of Formula (I) or a pharmaceutically acceptable salt thereof: 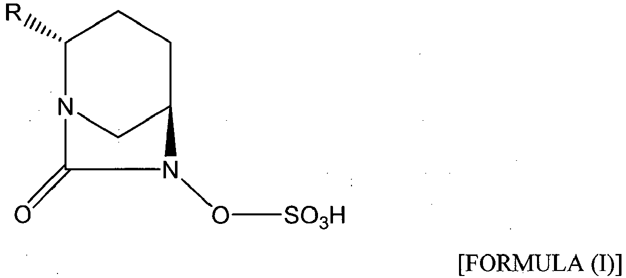 wherein
   ∘ R is selected from C(O)R¹ and C₁₋₄ alkyl, wherein the C₁₋₄ alkyl group is substituted with at least one halogen atom and is optionally further substituted with one or two substituents R²;
   ∘ R¹ is C₁₋₄ alkyl which is substituted with at least one halogen atom and is optionally further substituted with one or two substituents R²;
   ∘ each R² is independently selected from OH; C₁₋₄ alkoxy which is unsubstituted or substituted with one or more halogen atoms; C(O)R³; C(O)OH; C(O)OR³; 6- to 10-membered aryl; 5- to 6-membered heteroaryl; and 4- to 6-membered heterocyclyl; wherein the aryl, heteroaryl and heterocyclyl groups are unsubstituted or substituted with one, two or three substituents selected from halogen, OH, C(O)R³, C(O)OH, C(O)OR³ and C₁₋₄ alkyl and C₁₋₄ alkoxy groups which are themselves unsubstituted or substituted with one or more halogen atoms;
   ∘ R³ is C₁₋₄ alkyl which is unsubstituted or substituted with one or more halogen atoms.
2. A compound according to aspect 1, wherein R is selected from C(O)R¹ and C₁₋₄ alkyl, wherein the C₁₋₄ alkyl group is substituted with at least one halogen atom and is optionally further substituted with one substituent R².
3. A compound according to aspect 1, wherein R¹ is C₁₋₄ alkyl which is substituted with at least one halogen atom and is optionally further substituted with one substituent selected from OH and C₁₋₄ alkoxy which is itself unsubstituted or substituted with one or more halogen atoms.
4. A compound according to any one of the preceding aspects, wherein R¹ is C₁₋₂ alkyl, wherein the C₁₋₂ alkyl group is substituted with at least two halogen atoms selected from fluorine and chlorine.
5. A compound according to any one of the preceding aspects, wherein each R² is independently selected from OH; C₁₋₂ alkoxy which is itself unsubstituted or substituted with one or more halogen atoms; C(O)OR³, wherein R³ is unsubstituted C₁₋₂ alkyl; and unsubstituted 5- to 6-membered heteroaryl.
6. A compound according to any one of the preceding aspects, wherein each R² is independently selected from OH; OMe; C(O)OMe; and unsubstituted thiazolyl.
7. A compound according to aspect 1, wherein:
   - R is selected from C(O)R¹ and C₁₋₄ alkyl, wherein the C₁₋₄ alkyl group is substituted with at least one halogen atom and is optionally further substituted with one substituent R²;
   - R¹ is C₁₋₄ alkyl which is substituted with at least one halogen atom and is optionally further substituted with one substituent selected from OH and C₁₋₄ alkoxy, the C₁₋₄ alkyl group being unsubstituted or substituted with one or more halogen atoms; and
   - R² is selected from OH; C₁₋₂ alkoxy which is itself unsubstituted or substituted with one or more halogen atoms; C(O)OR³, wherein R³ is unsubstituted C₁₋₂ alkyl; and unsubstituted 5- to 6-membered heteroaryl.
8. A compound according to aspect 1, wherein:
   - R is selected from C(O)R¹ and C₁₋₄ alkyl, preferably C₁₋₂ alkyl, wherein the alkyl group is substituted with at least one halogen atom and is optionally further substituted with one substituent R²;
   - R¹ is C₁₋₂ alkyl, wherein the C₁₋₂ alkyl group is substituted with at least two halogen atoms selected from fluorine or chlorine; and
   - R² is selected from OH; OMe; C(O)OMe; and unsubstituted thiazolyl.
9. A compound according to any one of the preceding aspects, wherein R is C₁₋₂ alkyl, wherein the C₁₋₂ alkyl group is substituted with at least one halogen atom and is optionally further substituted with one substituent R².
10. A compound according to aspect 1, wherein R is selected from CF₃, CHF₂, CHCl₂, CCl₃, CH₂F, CF₂CH₃, CF₂CH₂CO₂Me, COCF₃, CF₂-thiazolyl, CF₂CH₂OCH₃, CF₂CH₂CH₂OH, CH(OH)CF₃, CH₂CF₃ and CF₂-oxetanyl.
11. A compound according to aspect 1, wherein R is selected from CF₃, CHF₂ and CHCl₂.
12. A compound according to any one of the preceding aspects, which compound is a sodium salt of a compound of Formula (I).
13. A pharmaceutical composition comprising a compound according to any one of the preceding aspects and a pharmaceutically acceptable carrier or diluent and optionally further comprising (i) an antibiotic agent and/or (ii) a metallo-β-lactamase inhibitor.
14. A combination of a compound according to any one of aspects 1 to 12 and one or more of (i) an antibiotic agent and (ii) a metallo-β-lactamase inhibitor.
15. A compound according to any one of aspects 1 to 12 for use in the treatment or prevention of bacterial infection by co-administration with an antibiotic agent and/or a metallo-β-lactamase inhibitor.
16. An antibiotic agent for use in the treatment or prevention of bacterial infection by co-administration with a compound according to any one of aspects 1 to 12, and optionally a metallo-β-lactamase inhibitor.
17. A metallo-β-lactamase inhibitor for use in the treatment or prevention of bacterial infection by co-administration with a compound according to any one of aspects 1 to 12, and optionally an antibiotic agent.
18. A composition according to aspect 13, a combination according to aspect 14 or a compound, antibiotic agent or metallo-β-lactamase inhibitor for use according to any one of aspects 15 to 17, wherein the antibiotic agent is a β-lactam antibiotic, preferably a β-lactam antibiotic selected from carbapenems, penicillins, cephalosporins and penems.
19. A composition, combination or compound, antibiotic agent or metallo-P-lactamase inhibitor for use according to aspect 18, wherein the antibiotic agent is a carbapenem antibiotic, preferably the antibiotic agent is meropenem.
20. A compound according to any one of aspects 1 to 12, or a composition or combination according to any one of aspects 13, 14, 18 or 19 for use in the removal or reduction of antibiotic resistance in Gram-negative bacteria.
21. A compound according to any one of aspects 1 to 12, or a composition or combination according to any one of aspects 13, 14, 18 or 19 for use in the treatment or prevention of bacterial infection.
22. A compound, antibiotic agent, metallo-β-lactamase inhibitor, composition or combination for use according to any one of aspects 15 to 21 wherein the Gram-negative bacteria are selected from Enterobacteriaceae, Pseudomonadaceae and Moraxellaceae, or the bacterial infection is caused by bacteria selected from Enterobacteriaceae, Pseudomonadaceae and Moraxellaceae.
23. A compound, antibiotic agent, metallo-β-lactamase inhibitor, composition or combination for use according to aspect 22 wherein the bacteria selected from Enterobacteriaceae, Pseudomonadaceae and Moraxellaceae are selected from *Klebsiella pneumoniae, Escherichia coli, Enterobacter Cloacae, Pseudomonas aeruginosa, Burkholderia cepacia* and *Acinetobacter baumannii.*
24. A compound, antibiotic agent, metallo-β-lactamase inhibitor, composition or combination for use according to aspect 22 wherein the bacterial infection is caused by Carbapenem Resistant Enterobacteriaceae.

### DETAILED DESCRIPTION OF THE INVENTION

### Definitions

As used herein, a C₁ to C₄ alkyl group is a linear or branched alkyl group containing from 1 to 4 carbon atoms. A C₁ to C₄ alkyl group is often a C₁ to C₃ alkyl group or a C₁ to C₂ alkyl group. Examples of C₁ to C₄ alkyl groups include methyl, ethyl, n-propyl, isopropyl, n-butyl, iso-butyl, sec-butyl, and tert-butyl. A C₁ to C₂ alkyl group is methyl or ethyl, typically methyl. For the avoidance of doubt, where two alkyl groups are present, the alkyl groups may be the same or different.

As used herein, a C₁₋₄ alkoxy is a C₁₋₄ alkyl group as defined above joined to an oxygen atom.

An alkyl or alkoxy group as used herein may be unsubstituted or substituted. Unless otherwise stated, substituted alkyl or alkoxy groups typically carry one or more, e.g. 1, 2, 3 or 4, such as one, two or three e.g. two, or three substituents. Preferred substituents are halogen atoms. Where indicated, an alkyl group may also be substituted with one or two groups R² as defined herein. The substituents on a substituted alkyl or alkoxy group are typically themselves unsubstituted unless otherwise stated. Where more than one substituent is present, these may be the same or different.

As used herein, a halogen is typically chlorine, fluorine, bromine or iodine and is preferably chlorine, bromine or fluorine, especially chorine or fluorine. Where a group or moiety is substituted with one or more halogen atoms, preferably it carries one, two, three or four halogen atoms, preferably two or three halogen atoms. Where a group or moiety is substituted with two or more halogen atoms, the halogen atoms may be the same or different. Typically, the halogen atoms are the same.

As used herein a 6- to 10-membered aryl group is a substituted or unsubstituted, monocyclic or fused polycyclic aromatic group containing from 6 to 10 carbon atoms in the ring portion. Examples include monocyclic groups such as phenyl and fused bicyclic groups such as naphthyl and indenyl. Phenyl (benzene) is preferred.

As used herein, a 5- to 6- membered heteroaryl group is a substituted or unsubstituted, monocyclic aromatic group containing 5 or 6 atoms in the ring portion, including at least one heteroatom, for example 1, 2 or 3 heteroatoms, typically selected from O, S and N. Examples of 5- and 6- membered heteroaryl groups include pyrrole, furan, thiophene, imidazole, oxazole, thiazole, pyridine, pyridazine, pyrimidine, and pyrazine.

As used herein, a 4- to 6- membered heterocyclyl group is a cyclic group containing from 4 to 6 atoms selected from C, O, N and S in the ring, including at least one heteroatom, and typically one or two heteroatoms. The heteroatom or heteroatoms are typically selected from O, N, and S. A heterocyclic group may be saturated or partially unsaturated. A 4- to 6- membered partially unsaturated heterocyclic group is a cyclic group containing from 4 to 6 atoms selected from C, O, N and S in the ring and containing 1 or 2, e.g. 1 double bond.

Examples of 4- to 6- membered saturated heterocyclic groups include oxetane, azetidine, piperazine, piperidine, morpholine, pyrrolidine, imidazolidine, and oxazolidine. Examples of 4- to 6- membered partially unsaturated heterocyclic groups include tetrahydropyrazine, tetrahydropyridine, dihydro-1,4-oxazine, tetrahydropyrimidine, dihydro-1,3-oxazine, dihydropyrrole, dihydroimidazole and dihydrooxazole.

An aryl, heterocyclyl or heteroaryl group may be unsubstituted or substituted as described herein. For example, an aryl, heterocyclyl or heteroaryl group may be unsubstituted or substituted with 1, 2 or 3, typically 1 or 2 such as e.g. 1 substituent. Suitable substituents include halogen, OH, C(O)C₁₋₄ alkyl, C(O)OH, C(O)OC₁₋₄ alkyl, C₁₋₄ alkyl and C₁₋₄ alkoxy, wherein the C₁₋₄ alkyl and C₁₋₄ alkoxy groups and moieties are themselves unsubstituted or substituted with one or more halogen atoms.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, benzoic, acetic, methanesulphonic, ethanesulphonic, benzenesulphonic or *p-*toluenesulphonic acid. Pharmaceutically acceptable bases include alkali metal (e.g. sodium or potassium) and alkaline earth metal (e.g. calcium or magnesium) hydroxides and organic bases such as alkyl amines, aralkyl amines and heterocyclic amines. Preferred pharmaceutically acceptable salts are salts formed at the SO₃H group with pharmaceutically acceptable bases, in particular quaternary ammonium salts e.g. tetrabutylammonium salts, or alkali metal salts, e.g. sodium or potassium salts, most preferably sodium salts.

In Formula (I), the bicyclic ring adopts the stereochemistry depicted. Thus, when the bicyclic ring is depicted in "chair" form (as below), the R group is in the axial "up" position, whilst the second ring carrying the OSO₃H substituent is in the axial "down" position.

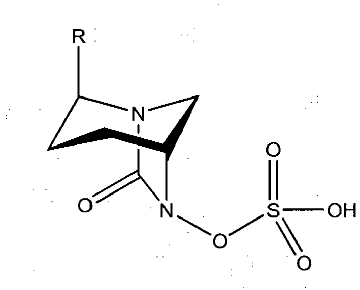

Typically, a compound or composition described herein contains at least 50%, preferably at least 60%, 75%, 90% or 95% of a compound of Formula (I) having the stereochemistry in the bicyclic ring (i.e. at the two chiral centres on the bicyclic ring) as depicted above. Preferably, the compound is substantially diastereomerically pure at the two chiral centres depicted above.

In Formula (I), one or more further chiral centres may be present in the R group. At these chiral centres, the stereochemistry is not limited and the compounds may be used in diastereomerically pure form, or as a mixture of isomers. Typically, a compound or composition described herein contains at least 50%, preferably at least 60, 75%, 90% or 95% of a compound according to Formula (I) which is diasteriomerically pure with regard to a chiral centre in the R group. Typically, a compound or composition of the invention comprises by weight at least 60%, such as at least 75%, 90%, or 95% of a single diastereomer. Preferably, the compound is substantially optically pure.

Further, for the avoidance of doubt, the compounds of the invention may be used in any tautomeric form.

As used herein, "removing or reducing resistance" to antibiotics, or "removing or reducing resistance" in bacteria, means that the bacterial resistance mechanism, e.g. the breakdown of an antibiotic compound by SBL, is prevented or inhibited. Therefore the effects of bacterial resistance (i.e. inefficacy of antibiotics) is removed or reduced. In other words, the compounds of the invention are useful in inhibiting or preventing hydrolysis of a β-lactam ring, i.e. in inhibiting or preventing hydrolysis of an antibiotic compound. They therefore improve the efficacy of antibiotics when used to treat infection caused by SBL-producing bacteria.

### Compounds of the Invention

Typically, in Formula (I), R is selected from C(O)R¹ and C₁₋₄ alkyl, wherein the C₁₋₄ alkyl group is substituted with at least one halogen atom (e.g. fluorine or chlorine) and is optionally further substituted with one substituent R². Preferably, R is selected from C(O)R¹ and C₁₋₂ alkyl, wherein the C₁₋₂ alkyl group is substituted with at least one halogen atom (e.g. fluorine or chlorine) and is optionally further substituted with one substituent R².

In one preferred aspect of the invention, R is C₁₋₄ alkyl, wherein the C₁₋₄ alkyl group is substituted with at least one halogen atom (e.g. fluorine or chlorine) and is optionally further substituted with one or two, e.g. one, substituent R². Preferably, the alkyl group is substituted with at least two, e.g. two or three, halogen atoms. The halogen atoms are preferably selected from fluorine and chlorine. Preferably, R is C₁₋₂ alkyl, wherein the C₁₋₂ alkyl group is substituted with at least one halogen atom (e.g. fluorine or chlorine) and is optionally further substituted with one substituent R². More preferably, R is C₁₋₂ alkyl, wherein the C₁₋₂ alkyl group is substituted with at least two halogen atoms selected from fluorine and chlorine. Most preferably R is a methyl group which is substituted with two or three halogen atoms selected from fluorine and chlorine, e.g. CF₃, CCl₃, CHF₂ and CHCl₂.

Where R represents C(O)R¹, typically R¹ is C₁₋₄ alkyl which is substituted with at least one halogen atom (e.g. fluorine or chlorine) and is optionally further substituted with one substituent selected from OH and C₁₋₄ alkoxy which is itself unsubstituted or substituted with one or more halogen atoms (e.g. fluorine or chlorine). Preferably, R¹ is C₁₋₄ alkyl which is substituted with at least one, more preferably at least two (e.g. two or three), halogen atoms. The halogen atoms are preferably selected from fluorine and chlorine. More preferably R¹ is C₁₋₂ alkyl, wherein the C₁₋₂ alkyl group is substituted with at least two halogen atoms selected from fluorine and chlorine. Most preferably R¹ is a methyl group which is substituted with two or three halogen atoms selected from fluorine and chlorine, e.g. CF₃.

When R² represents 6- to 10-membered aryl, it is typically phenyl. When R² represents 5-to 6-membered heteroaryl, it is typically a 5-membered heteroaryl selected from thiazolyl, pyrrolyl, furanyl, thiophenyl, imidazolyl and oxazolyl, preferably thiazolyl. When R² represents 4- to 6-membered heterocyclyl, it is typically a 4- or 5-membered heterocyclyl group selected from oxetanyl, azetidinyl, tetrahydrofuranyl, pyrrolidinyl and tetrahydrothiophenyl. Preferably it is a 4-membered heterocyclyl group selected from oxetanyl and azetidinyl, most preferably oxetanyl.

When R² represents an aryl, heteroaryl or heterocyclyl group, it is unsubstituted or substituted with one, two or three (e.g. one or two) substituents selected from halogen, OH, C(O)R³, C(O)OH, C(O)OR³ and C₁₋₄ alkyl and C₁₋₄ alkoxy groups which are themselves unsubstituted or substituted with one or more halogen atoms. Preferred substituents are halogen; OH; and C₁₋₂ alkyl and C₁₋₂ alkoxy groups which are themselves unsubstituted or substituted with one or more halogen atoms. More preferred substituents are halogen, OH, Me and OMe. Most preferably when R² represents an aryl, heteroaryl or heterocyclyl group, it is unsubstituted.

Each R² is typically independently selected from OH; C₁₋₂ alkoxy which is itself unsubstituted or substituted with one or more halogen atoms; C(O)OR³, wherein R³ is unsubstituted C₁₋₂ alkyl; and unsubstituted 5- to 6-membered heteroaryl. Preferably each R² is independently selected from OH; OMe; C(O)OMe; and unsubstituted thiazolyl.

R³ is typically unsubstituted C₁₋₂ alkyl, preferably methyl.

Preferred compounds of the invention are those wherein:
- R is selected from C(O)R¹ and C₁₋₄ alkyl, wherein the C₁₋₄ alkyl group is substituted with at least one halogen atom (e.g. fluorine or chlorine) and is optionally further substituted with one substituent R²;
- R¹ is C₁₋₄ alkyl which is substituted with at least one halogen atom (e.g. fluorine or chlorine) and is optionally further substituted with one substituent selected from OH and C₁₋₄ alkoxy which is itself unsubstituted or substituted with one or more halogen atoms (e.g. fluorine or chlorine); and
- R² is selected from OH; C₁₋₂ alkoxy which is itself unsubstituted or substituted with one or more halogen atoms; C(O)OR³, wherein R³ is unsubstituted C₁₋₂ alkyl; and unsubstituted 5- to 6-membered heteroaryl.

More preferred compounds of the invention are those wherein:
- R is selected from C(O)R¹ and C₁₋₄ alkyl, in particular C₁₋₂ alkyl, wherein the alkyl group is substituted with at least one halogen atom (e.g. fluorine or chlorine) and is optionally further substituted with one substituent R²;
- R¹ is C₁₋₂ alkyl, wherein the C₁₋₂ alkyl group is substituted with at least two halogen atoms selected from fluorine and chlorine; and
- R² is selected from OH; OMe; C(O)OMe; and unsubstituted thiazolyl.

Most preferred compounds of the invention are those wherein R is COCF₃ or C₁₋₂ alkyl, wherein the C₁₋₂ alkyl group is substituted with at least one halogen atom (e.g. fluorine or chlorine) and is optionally further substituted with one substituent R², wherein R² is selected from OH; OMe; C(O)OMe; and unsubstituted thiazolyl. More preferably, R is C₁₋₂ alkyl, wherein the C₁₋₂ alkyl group is substituted with at least two halogen atoms selected from fluorine or chlorine. Most preferably R is a methyl group which is substituted with two or three halogen atoms selected from fluorine and chlorine, e.g. CF3, CCl₃, CHF₂ and CHCl₂.

Preferred examples of R groups include CF₃, CHF₂, CHCl₂, CCl₃, CH₂F, CF₂CH₃, CF₂CH₂CO₂Me, COCF₃, CF₂-thiazolyl, CF₂CH₂OCH₃, CF₂CH₂CH₂OH, CH(OH)CF₃, CH₂CF₃, CF₂-oxetanyl, in particular CF₃, CHF₂ and CHCl₂.

Preferred compounds of the invention include:
(2S, 5R)-7-oxo-2-(trifluoromethyl)-1,6-diazabicyclo[3.2.1]octan-6-yl hydrogen sulfate;
(2S, 5R)-7-oxo-2-(difluoromethyl)-1,6-diazabicyclo[3.2.1]octan-6-yl hydrogen sulfate;
(2S,5R)-2-(dichloromethyl)-7-oxo-1,6-diazabicyclo[3.2.1]octan-6-yl hydrogen sulfate;
(2S, 5R)-7-oxo-2-(trichloromethyl)-1,6-diazabicyclo[3.2.1]octan-6-yl hydrogen sulfate;
(2S, 5R)-2-(fluoromethyl)-7-oxo-1,6-diazabicyclo[3.2.1]octan-6-yl hydrogen sulfate;
(2S, 5R)-7-oxo-2-(trichloromethyl)-1,6-diazabicyclo[3.2.1]octan-6-yl hydrogen sulfate;
(2S, 5R)-2-(1,1-difluoroethyl)-7-oxo-1,6-diazabicyclo[3.2.1]octan-6-yl hydrogen sulfate;
Methyl 3,3-difluoro-3-((2S, 5R)-7-oxo-6-(sulfooxy)-1,6-diazabicyclo[3.2.1]octan-2-yl)propanoate;
(2S, 5R)-7-oxo-2-(2,2,2-trifluoroacetyl)-1,6-diazabicyclo[3.2.1]octan-6-yl hydrogen sulfate;
(2S, 5R)-2-[difluoro(1,3-thiazol-2-yl)methyl]-7-oxo-1,6-diazabicyclo[3.2.1]octan-6-yl hydrogen sulfate;
(2S, 5R)-2-(1,1-difluoro-2-methoxyethyl)-7-oxo-1,6-diazabicyclo[3.2.1]octan-6-yl hydrogen sulfate;
(2S, 5R)-2-(1,1-difluoro-2-hydroxyethyl)-7-oxo-1,6-diazabicyclo[3.2.1]octan-6-yl hydrogen sulfate;
(2S, 5R)-7-oxo-2-(2,2,2-trifluoro-1-hydroxyethyl)-1,6-diazabicyclo[3.2.1]octan-6-yl hydrogen sulfate;
(2S, 5R)-7-oxo-2-(2,2,2-trifluoroethyl)-1,6-diazabicyclo[3.2.1]octan-6-yl hydrogen sulfate; and
(2S, 5R)-2-[difluoro(oxetan-3-yl)methyl]-7-oxo-1,6-diazabicyclo[3.2.1]octan-6-yl hydrogen sulfate;
and pharmaceutically acceptable salts thereof.

Preferred pharmaceutically acceptable salts of these compounds are salts at the SO₃H group, in particular quaternary ammonium salts, e.g. tetrabutylammonium salts, and alkali metal salts, e.g. sodium and potassium salts. Sodium salts as set out below are most preferred:
Sodium (2S, 5R)-7-oxo-2-(trifluoromethyl)-1,6-diazabicyclo[3.2.1]octan-6-yl sulfate;
Sodium (2S, 5R)-7-oxo-2-(difluoromethyl)-1,6-diazabicyclo[3.2.1]octan-6-yl sulfate;
Sodium (2S,5R)-2-(dichloromethyl)-7-oxo-1,6-diazabicyclo[3.2.1]octan-6-yl sulfate;
Sodium (2S, 5R)-7-oxo-2-(trichloromethyl)-1,6-diazabicyclo[3.2.1]octan-6-yl sulfate;
Sodium (2S, 5R)-2-(fluoromethyl)-7-oxo-1,6-diazabicyclo[3.2.1]octan-6-yl sulfate;
Sodium (2S, 5R)-7-oxo-2-(trichloromethyl)-1,6-diazabicyclo[3.2.1]octan-6-yl sulfate;
Sodium (2S, 5R)-2-(1,1-difluoroethyl)-7-oxo-1,6-diazabicyclo[3.2.1]octan-6-yl sulfate;
Methyl (2S, 5R)-3,3-difluoro-3-(7-oxo-6-{[(sodiooxy)sulfonyl]oxy}-1,6-diazabicyclo[3.2.1]octan-2-yl)propanoate;
Sodium (2S, 5R)-7-oxo-2-(2,2,2-trifluoroacetyl)-1,6-diazabicyclo[3.2.1]octan-6-yl sulfate; Sodium (2S, 5R)-2-[difluoro(1,3-thiazol-2-yl)methyl]-7-oxo-1,6-diazabicyclo[3.2.1]octan-6-yl sulfate;
Sodium (2S, 5R)-2-(1,1-difluoro-2-methoxyethyl)-7-oxo-1,6-diazabicyclo[3.2.1]octan-6-yl sulfate;
Sodium (2S, 5R)-2-(1,1-difluoro-2-hydroxyethyl)-7-oxo-1,6-diazabicyclo[3.2.1]octan-6-yl sulfate;
Sodium (2S, 5R)-7-oxo-2-(2,2,2-trifluoro-1-hydroxyethyl)-1,6-diazabicyclo[3.2.1]octan-6-yl sulfate;
Sodium (2S, 5R)-7-oxo-2-(2,2,2-trifluoroethyl)-1,6-diazabicyclo[3.2.1]octan-6-yl sulfate; and
Sodium (2S, 5R)-2-[difluoro(oxetan-3-yl)methyl]-7-oxo-1,6-diazabicyclo[3.2.1]octan-6-yl sulfate.

Most preferred compounds are:
(2S, 5R)-7-oxo-2-(trifluoromethyl)-1,6-diazabicyclo[3.2.1]octan-6-yl hydrogen sulfate;
(2S, 5R)-7-oxo-2-(difluoromethyl)-1,6-diazabicyclo[3.2.1]octan-6-yl hydrogen sulfate; and
(2S,5R)-2-(dichloromethyl)-7-oxo-1,6-diazabicyclo[3.2.1]octan-6-yl hydrogen sulfate;
and pharmaceutically acceptable salts thereof.

Preferred pharmaceutically acceptable salts of these compounds are salts at the SO₃H group, in particular quaternary ammonium salts, e.g. tetrabutylammonium salts, and alkali metal salts, e.g. sodium and potassium salts. Sodium salts as set out below are most preferred:
Sodium (2S, 5R)-7-oxo-2-(trifluoromethyl)-1,6-diazabicyclo[3.2.1]octan-6-yl sulfate;
Sodium (2S, 5R)-7-oxo-2-(difluoromethyl)-1,6-diazabicyclo[3.2.1]octan-6-yl sulfate; and
Sodium (2S,5R)-2-(dichloromethyl)-7-oxo-1,6-diazabicyclo[3.2.1]octan-6-yl sulfate.

### Synthesis

The compounds of the invention can be synthesised according to the following Scheme A:

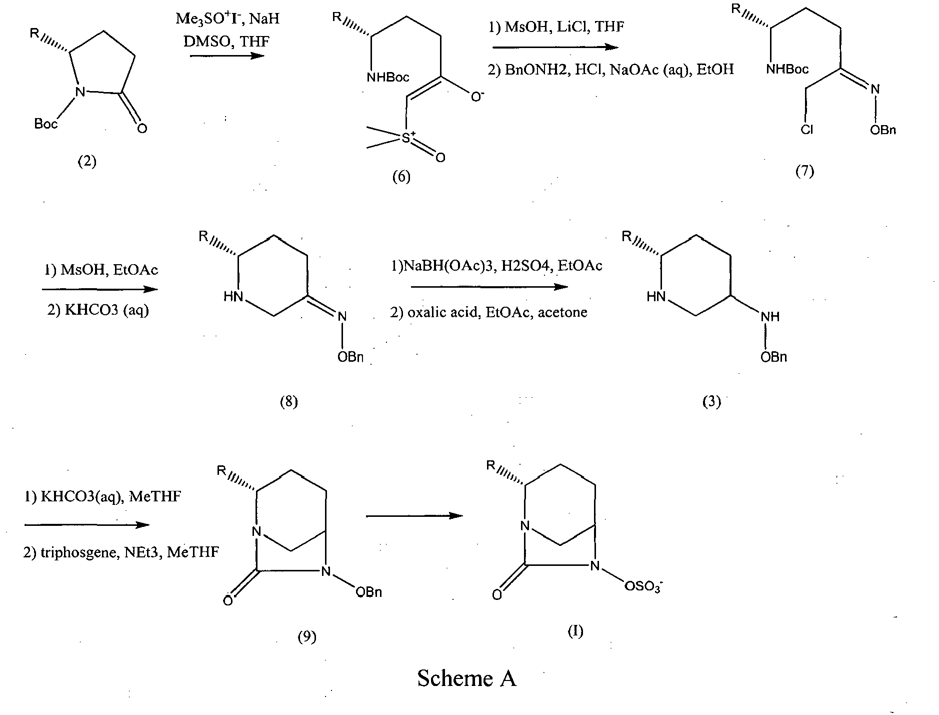

The BOC-pyrrolidinone **(2)** may be reacted with a sulfoxonium ylid reagent to give *in situ* the new ring-opened ylid **(6).** Protonation generates a positively charged sulfoxonium species which is nucleophilically attacked by chloride anion, resulting in loss of DMSO and generating the chloromethyl ketone which condenses with O-benzyl hydroxylamine to give oxime **(7).** This need not be isolated but may be treated with acid to remove the BOC protecting group and on basification spontaneous cyclisation occurs with formal loss of HCl to give ring expanded **(8).** Once again this need not be isolated but stereoselective reduction of the oxime double bond followed by salt formation with oxalic acid facilitates isolation of piperidine **(3).** Cyclisation using triphosgene as a phosgene source generates the bicyclic **urea (9)** then debenzylation and sulfonation produces the compound of formula (I). The R group may be protected using suitable protecting means, as required. Further details regarding synthetic routes, and alternative synthetic schemes, to provide the compounds of the invention are set out in the general synthetic methodology section below.

### Compositions and Combinations

The compounds of the invention may be provided in a pharmaceutical composition, the pharmaceutical composition comprising a compound of the invention together with a pharmaceutically acceptable carrier or diluent. Typically, the composition contains up to 85 wt% of a compound of the invention. More typically, it contains up to 50 wt% of a compound of the invention. Preferred pharmaceutical compositions are sterile and pyrogen free. Further, when the pharmaceutical compositions provided by the invention contain a compound of the invention which is optically active, the compound of the invention is typically a substantially pure optical isomer.

As explained above, the compounds of the invention are useful in treating or preventing bacterial infection. In particular, they are inhibitors of SBL enzymes and are therefore useful for removing or reducing resistance of Gram-negative bacteria to antibiotics. The compounds may be used alone or they may be used in combination therapies with antibiotic agents, to enhance the action of the antibiotic agent. The compounds may also be used in combination with MBL inhibitors, in particular where the bacteria causing the infection are resistant to treatment by an antibiotic agent alone and the resistance is, or is suspected of being, caused at least in part by metallo-β-lactamase enzymes.

The present invention therefore also provides a combination of (i) a compound of the invention; and one or more of (ii) a metallo-β-lactamase (MBL) inhibitor; and (iii) an antibiotic agent. The combinations may also comprise further active agents if desired.

In the combinations of the invention, the active agents may each be provided in a single formulation or one or more of them may be separately formulated. Where separately formulated, the two or more agents may be administered simultaneously or separately. Active agents which are separately formulated may be provided in the form of a kit, optionally together with instructions for their administration. For example, the kit may comprise (i) a composition comprising a compound of the invention; and one or both of (ii) a composition comprising an antibiotic agent and (iii) a composition comprising an MBL inhibitor, optionally together with instructions for their use. Where two or more active agents are formulated together, the two or more active agents may be provided as a pharmaceutical composition. The pharmaceutical composition may therefore comprise (i) a compound of the invention as described herein; (ii) a pharmaceutically acceptable carrier or diluent; and optionally one or both of (iii) an antibiotic agent; and (iv) a metallo-β-lactamase (MBL) inhibitor.

Thus, the compound of the invention and the antibiotic agent may be formulated together or separately. Further, the compound of the invention and the MBL inhibitor may be formulated together or separately. Where all three active agents are present, the compound of the invention, the MBL inhibitor and the antibiotic agent may each be provided in a single formulation, or they may be separately formulated. Alternatively, two of the components may be provided in a single formulation and the remaining component may be provided separately. In other words, the compound of the invention may be formulated with the MBL inhibitor and the antibiotic agent; or the compound of the invention may be formulated with the MBL inhibitor whilst the antibiotic agent is provided separately; or the compound of the invention may be formulated with the antibiotic agent whilst the MBL inhibitor is provided separately; or the MBL inhibitor may be formulated with the antibiotic agent whilst the compound of the invention is provided separately; or the compound of the invention, the MBL inhibitor and the antibiotic agent may each be formulated separately.

Preferably, the antibiotic agent which is administered with the compound of the invention, or used in the combinations or compositions of the invention, is a β-lactam antibiotic. More preferably, the antibiotic agent is a β-lactam antibiotic selected from carbapenems, penicillins, cephalosporins and penems. Examples of carbapenem antibiotics include Benapenem, Imipenem, Meropenem, Ertapenem, Doripenem and Biapenem. Examples of penicillins include Amoxicillin, Ampicillin, Ticarcillin, Piperacillin and Cloxacillin. Examples of cephalosporins include Cefepime, Cefazolin, Ceftriaxone, Ceftazidine and Ceftobiprole. Examples of penems include Faropenem. Other antibiotic agents include tobramycin, neomycin, streptomycin, gentamycin, tazobactam, rifampicin, ciprofloxacin, amikacin, colistin, aztreonam and levofloxacin. Preferably, the β-lactam antibiotic is a carbapenem antibiotic, more preferably imipenem or meropenem, most preferably meropenem.

Where an MBL inhibitor is present in the combinations or compositions of the invention, or is administered with a compound of the invention, the MBL inhibitor is preferably a compound as described in WO 2014/198849, GB2533136, PCT/EP2018/069827 or EP18290056.3.

The compounds, compositions or combinations described herein may be administered in a variety of dosage forms. Thus, they can be administered orally, for example as tablets, troches, lozenges, aqueous or oily suspensions, dispersible powders or granules. They may also be administered parenterally, whether subcutaneously, intravenously, intramuscularly, intrasternally, transdermally or by infusion techniques. The compound, composition or combination may also be administered as a suppository. Preferably, the compound, composition or combination is administered via parenteral administration, in particular via intravenous administration.

The compounds, compositions or combinations described herein are typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. Suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for injection may contain as carrier, for example, sterile water or preferably they may be in the form of sterile, aqueous, isotonic saline solutions. Pharmaceutical compositions suitable for delivery by needleless injection, for example, transdermally, may also be used.

### Therapeutic Efficacy

The compounds of the present invention are therapeutically useful. The present invention therefore provides compounds, compositions and combinations as described herein, for use in medicine. The present invention provides compounds as described herein, for use in treating the human or animal body. For the avoidance of doubt, the compound of the invention may be administered in the form of a solvate.

The compounds, compositions and combinations of the invention are useful in treating or preventing bacterial infection. The present invention therefore provides a compound, composition or combination of the invention for use in treating or preventing bacterial infection. The invention also provides the use of a compound, composition or combination of the invention in the manufacture of a medicament for use in the prevention or treatment of bacterial infection. The invention also provides a method of treating or preventing bacterial infection in a subject in need thereof, which method comprises administering to said subject an effective amount of a compound, composition or combination as described herein.

The compounds of the invention may be used as standalone therapeutic agents. For example, the compounds of the invention may be used as standalone adjuncts in antibacterial therapy, for example in chemotherapy regimes. Alternatively, they may be used in combination with antibiotic agents, and optionally with MBL inhibitors, to enhance the action of the antibiotic agent. The compounds of the invention may find particular use in treating or preventing bacterial infection caused by bacteria which are resistant to treatment with antibiotic agents when administered alone, particularly where the resistance is caused by presence of SBL enzymes. Treatment or prevention of such infection with β-lactam antibiotics alone may be unsuccessful. The compounds are therefore useful in the removal or reduction of antibiotic resistance, in particular in Gram-negative bacteria. In particular, the compounds are useful in removing or reducing resistance caused by SBL enzymes.

The invention also provides a compound of the invention for use in treating or preventing bacterial infection by co-administration with (i) an antibiotic agent and/or (ii) an MBL inhibitor. Also provided is an antibiotic agent for use in treating or preventing bacterial infection by co-administration with (i) a compound of the invention and optionally (ii) an MBL inhibitor. Also provided is an MBL inhibitor for use in treating or preventing bacterial infection by co-administration with (i) a compound of the invention and optionally (ii) an antibiotic agent.

Also provided is the use of a compound of the invention in the manufacture of a medicament for use in treating or preventing bacterial infection by co-administration with (i) an antibiotic agent and/or (ii) an MBL inhibitor. Also provided is the use of an antibiotic agent in the manufacture of a medicament for use in treating or preventing bacterial infection by co-administration with (i) a compound of the invention and optionally (ii) an MBL inhibitor. Also provided is the use of an MBL inhibitor in the manufacture of a medicament for use in treating or preventing bacterial infection by co-administration with (i) a compound of the invention and optionally (ii) an antibiotic agent.

Also provided is a method of treating or preventing bacterial infection in a subject in need thereof, which method comprises co-administering a compound of the invention with (i) and antibiotic agent and/or (ii) an MBL inhibitor.

In one aspect, the subject to be treated is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters. The subject can be any animal that is capable of being infected by a bacterium.

The compounds, compositions and combinations described herein are useful in the treatment of bacterial infection which occurs after a relapse following an antibiotic treatment. The compounds, compositions and combinations can therefore be used in the treatment of a patient who has previously received antibiotic treatment for the (same episode of) bacterial infection.

The bacterium causing the infection may be any bacterium expressing an SBL enzyme or an analogue thereof. Typically the bacterium causing the infection expresses an SBL enzyme. The bacterium is typically Gram-negative. The bacterium may in particular be a pathogenic bacterium. Typically, the bacterial infection to be treated using the compounds of the invention is resistant to treatment with a conventional antibiotic when the conventional antibiotic is used alone or in a combination with another partner. For example, the bacterial infection to be treated using the compounds of the invention may be resistant to treatment when the conventional antibiotic is used solely in combination with an MBL inhibitor.

The Gram-negative bacteria of which antibiotic resistance can be removed using the compounds of general formula (I) are bacteria which produce serine-β-lactamases, which may be serine-β-lactamases of subclasses A, C or D. In particular, the bacteria may be those which produce extended spectrum β-lactamases (ESBLs) and/or carbapenemases, in particular the OXA class of carbapenemases.

The bacterial infection may be caused by bacteria from the families Enterobacteriaceae, Pseudomonadaceae and/or Moraxellaceae, more typically the bacterial infection is caused by bacteria from the families Enterobacteriaceae and/or Pseudomonadaceae, and most typically the bacterial infection is caused by bacteria from the family Enterobacteriaceae. The bacterial infection may be caused by *Pseudomonas* (e.g. *Pseudomonas aeruginosa, Pseudomonas oryzihabitans,* or *Pseudomonas plecoglossicida*), *Klebsiella, Escherichia, Enterobacter, Acinetobacter* or *Burkholderia.* For example, the bacterial infection may be caused by *Klebsiella pneumoniae, Escherichia coli, Enterobacter Cloacae, Pseudomonas aeruginosa, Burkholderia cepacia* or *Acinetobacter baumannii.* The bacterium may be an opportunistic pathogen.

The compound, composition or combination of the invention may be used to treat or prevent infections and conditions caused by any one or a combination of the above-mentioned bacteria. In particular, the compound, composition or combination of the invention may be used in the treatment or prevention of pneumonia. The compound, composition or combination may also be used in the treatment of septic shock, urinary tract infection, and infections of the gastrointestinal tract, skin or soft tissue.

The compound, composition or combination of the invention may be used to treat patients with Carbapenem Resistant Enterobacteriaceae (CRE). CRE can be found in the community or in hospitals and other institutions which are commonly associated with long term patients and those that are undergoing significant medical interventions such as are commonly cared for in Intensive Care Units (ICUs).

A compound, composition or combination of the invention can be administered to the subject in order to prevent the onset or reoccurrence of one or more symptoms of the bacterial infection. This is prophylaxis. In this embodiment, the subject can be asymptomatic. The subject is typically one that has been exposed to the bacterium. A prophylactically effective amount of the agent or formulation is administered to such a subject. A prophylactically effective amount is an amount which prevents the onset of one or more symptoms of the bacterial infection.

A compound, composition or combination of the invention can be administered to the subject in order to treat one or more symptoms of the bacterial infection. In this embodiment, the subject is typically symptomatic. A therapeutically effective amount of the agent or formulation is administered to such a subject. A therapeutically effective amount is an amount effective to ameliorate one or more symptoms of the disorder.

A therapeutically or prophylactically effective amount of the compound of the invention is administered to a subject. The dose may be determined according to various parameters, especially according to the compound used; the age, weight and condition of the subject to be treated; the route of administration; and the required regimen. A physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific inhibitor, the age, weight and conditions of the subject to be treated, the type and severity of the disease and the frequency and route of administration. Preferably, daily dosage levels are from 70 mg to 3.5 g.

When the compound of the invention is administered to a subject in combination with another active agent (for example in the form of a pharmaceutical combination comprising an antibiotic agent and optionally an MBL inhibitor), the dose of the other active agent (e.g. MBL inhibitor and/or antibiotic agent) can be determined as described above. The dose may be determined according to various parameters, especially according to the agent used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific inhibitor, the age, weight and conditions of the subject to be treated, the type and severity of the disease and the frequency and route of administration. Preferably, daily dosage levels are from 70 mg to 3.5 g.

The following Examples illustrate the invention. They do not however, limit the invention in any way. In this regard, it is important to understand that the particular assay used in the Examples section is designed only to provide an indication of biological activity. There are many assays available to determine biological activity, and a negative result in any one particular assay is therefore not determinative.

### Experimental Details

### General synthetic methodology

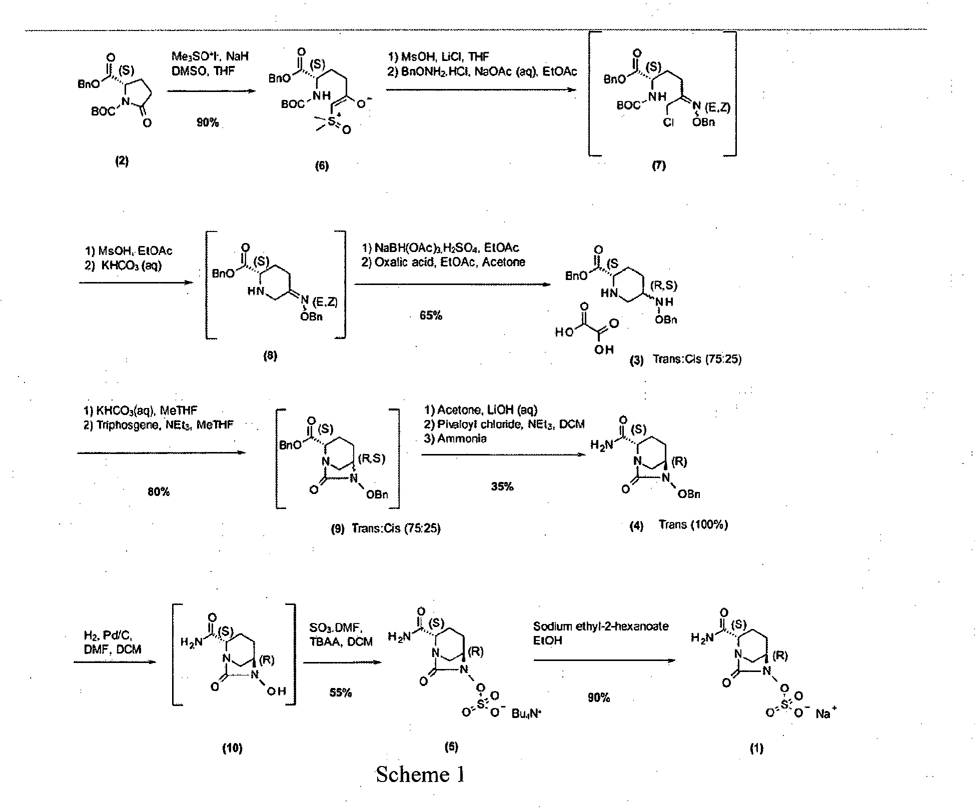

There are a number of approaches to the synthesis of avibactam **(1).** One of the major routes is shown in Scheme 1, reproduced from Ball, M. et al, Organic Process Research and Development, 2016, 20, 1799. The BOC-pyrrolidinone **(2)** reacts with sulfoxonium ylid reagent to give *in situ* the new ring-opened ylid **(6).** Protonation generates a positively charged sulfoxonium species which is nucleophilically attacked by chloride anion, resulting in loss of DMSO and generating the chloromethyl ketone which condenses with O-benzyl hydroxylamine to give oxime **(7).** Again, this is not isolated but treated with acid to remove the BOC protecting group and on basification spontaneous cyclisation occurs with formal loss of HCl to give ring expanded **(8).** Once again this is not isolated but stereoselective reduction of the oxime double bond followed by salt formation with oxalic acid facilitates isolation of piperidine **(3).** Cyclisation using triphosgene as a phosgene source generates the bicyclic urea **(9)** then ester hydrolysis and amide formation generates the primary carboxamide of avibactam as in **(4).** Finally debenzylation and sulfonation produces avibactam **(1).**

Use of substituents other than ester on the starting pyrrolidinone can access alternative analogues of avibactam. Furthermore, intermediate **(3)** is now commercially available (eg from Shanghai Habo Chemical Technology Company or Frapps Chemicals Co, Zhejiang, China, http://www.frappschem.com), so a variety of substituents can be accessed from this later stage intermediate. Examples are shown in schemes 2a and b below.

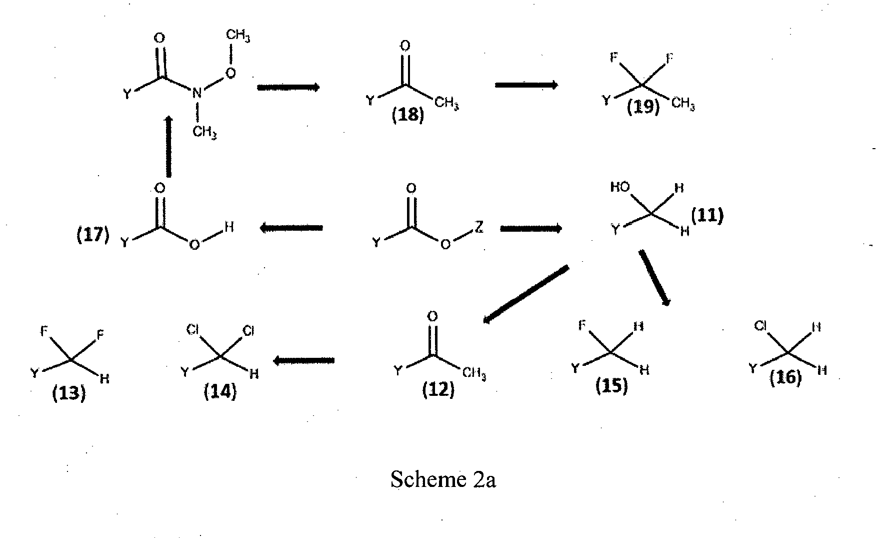

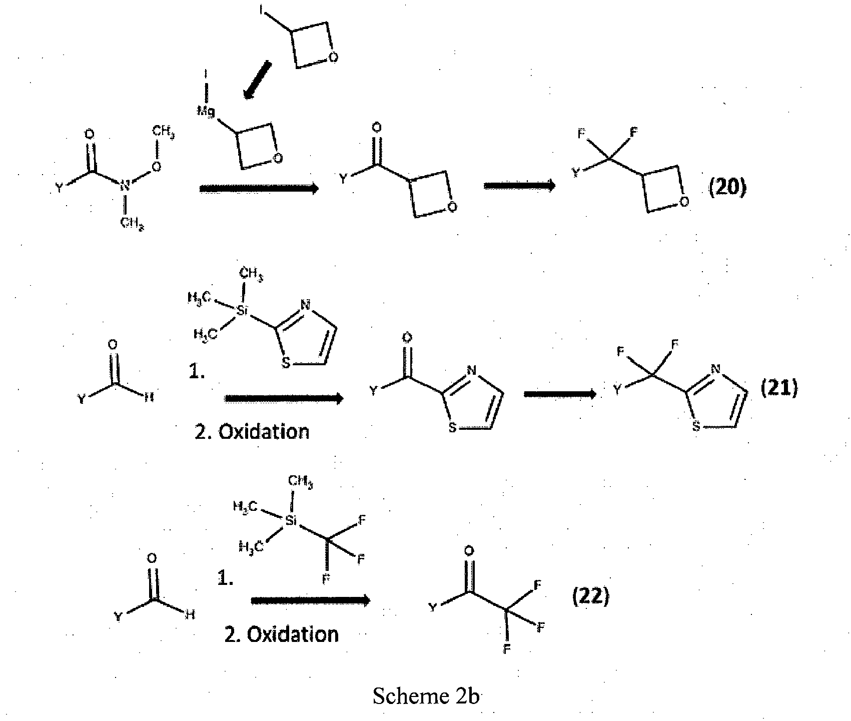

In Schemes 2a and 2b, Z represents a suitable ester substituent such as the benzyl group in (9) of Scheme 1. Y represents the group: 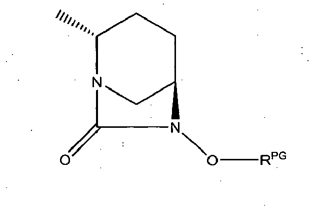 where R^{PG} represents a protecting group, for example a benzyl or -CH₂CH=CH₂ group. This protecting group can be removed after synthesis of the remainder of the compound, and the SO₃H or SO₃⁻ group provided, in a similar manner to that depicted in Scheme 1 above.

As depicted in Scheme 2a, the ester functionality such as **(9)** in Scheme 1 can be reduced to an alcohol **(11)** which can be oxidised to an aldehyde **(12).** The aldehyde can be transformed into a difluoromethyl substituent **(13)** or a dichloromethyl substituent **(14)** using DAST (diethylaminosulfur trifluoride, see Xu, Y., Prestwich, G. D., Journal of Organic Chemistry, 2002, 67, 20, 7158 - 7161) or PC15 (Gauthier, J. et al, Bioorganic and Medicinal Chemistry Letters, 2008, 18, 923 - 928) or similar reagents, chemistry that is well known to those skilled in the art. Similarly the alcohol can be transformed into fluoromethyl **(15)** with DAST (Liu, Y. et al, Organic Letters, 2004, 6, 209 - 212) and chloromethyl **(16)** with thionyl chloride (Gudipati, V. et al, Journal of Organic Chemistry, 2006, 71, 3599 - 3607). Also the acid **(17)** can be converted to methyl ketone **(18)** using standard Weinreb amide chemistry which can be similarly converted into the difluoroethyl substituent **(19)** with DAST.

A wide range of substituents can be accessed using Weinreb amide chemistry as shown in Scheme 2b. For instance, the Grignard derived from 3-iodooxetane (commercially-available from Sigma Aldrich) can in principle react with the aforementioned Weinreb reagent to produce the analogous ketone which can be converted to the difluro analogue **(20)** using the usual DAST reagent. This chemistry can also be applied to longer chain alkyldifluoromethylene substituents as well as aryldifluoromethylene substituents.

For certain specific heterocycles, alternative chemistry may be used. For instance 2-trimethylthiazole can react with aldehydes followed by TMS ether cleavage and oxidation of the alcohol to generate the 2-thiazolyl ketone **(21)** (for an example of this chemistry, see Dondoni, A. *et al*, JOC2004, **69,** 5023) which can be transformed into the difluoromethylene using DAST reagent. Related silicon chemistry can be used to generate for example the trifluoromethyl ketone substituent, as (trifluoromethyl)trimethylsilane can react with aldehydes to give the corresponding carbinol (Cheng, H., et al Tet. Lett., 2013, 54, 4483). Standard oxidation then accesses the trifluoromethyl ketone **(22).**

### Examples

### General Techniques

1H NMR spectra are reported at 300 or 400 MHz in DMSO-d6 solutions (δ in ppm), using chloroform as the reference standard (7.25 ppm). When peak multiplicities are reported, the following abbreviations are used: s (singlet), d (doublet), t (triplet), m (multiplet), bs (broadened singlet), dd (doublet of doublets), dt (doublet of triplets), q (quartet). Coupling constants, when given, are reported in hertz (Hz).

All reactions were conducted under an inert atmosphere of nitrogen or argon, unless stipulated (eg hydrogenations reactions).

### Abbreviations

- ACN: acetonitrile
- DAST: diethylaminosulfur trifluoride
- DCM: dichloromethane
- DIPEA: N,N-di*iso*propylethylamine
- DMAP: 4-dimethylaminopyridine
- DMSO: dimethylsulfoxide
- h: hours
- HMBC: heteronuclear multiple bond correlation
- p.s.i: pounds per square inch
- TEA: triethylamine
- TEMPO: (2,2,6,6-tetramethylpiperidin-1-yl)oxyl
- THF: tetrahydrofuran

### Example 1

### Sodium (2S, 5R)-7-oxo-2-(trifluoromethyl)-1,6-diazabicyclo[3.2.1]octan-6-yl sulfate

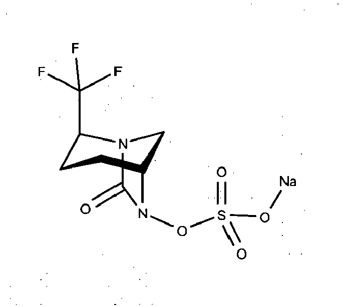

This was prepared using essentially the same methodology as reported for avibactam (Ball, M. et al, Organic Process Research and Development, 2016, 20, 1799) except starting from (5S)-5-(trifluoromethyl)-2-pyrrolidinone.

### a. tert-Butyl (5S)-2-oxo-5-(trifluoromethyl)pyrrolidine-1-carboxylate

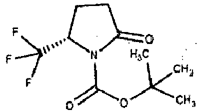

A solution of (5S)-5-(trifluoromethyl)-2-pyrrolidinone (commercially available from Manchester Organics) (5g, 32.7 mmol) in DCM (60 mL) at 0°C was treated with triethylamine (5.5 mL, 4.0g, 39.2 mmol) and DMAP (0.4g, 0.1 mmol) then a solution of di-tert butyl dicarbonate (8.6g, 39.2 mmol) in DCM (20 mL) was added dropwise over 10 minutes. After 0.5h the mixture was partitioned between DCM and 10% aqueous citric acid and the organic extract washed with water, dried (Na2SO4) and evaporated affording an oil (9.4g) which was chromatographed on silica eluting with 0-25% ethyl acetate in toluene affording an oil (7.9g, 96%).
M/z 276 (M+Na)⁺

### b. (3R,6S)-N-(Benzyloxy)-6-(trifluoromethyl)piperidin-3-amine

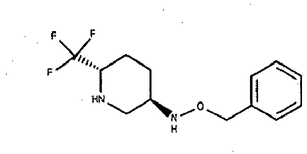

For a detailed explanation of this reaction sequence see (Ball, M. et al, Organic Process Research and Development, 2016, 20, 1799).

DMSO (10 mL) was added to a mixture of trimethylsulfoxonium iodide (1.6g, 7.3 mmol) and potassium t-butoxide (0.72g, 6.4 mmol) in THF (7 mL). The mixture was stirred at room temperature for 1h then cooled to -12°C (internal temperature). A solution of tert-butyl (5S)-2-oxo-5-(trifluoromethyl)pyrrolidine-1-carboxylate (1.5g, 5.8 mmol) in THF (4 mL) was added dropwise over 5 minutes and the mixture stirred at -12°C for 1h. The reaction mixture was treated with 20% aqueous ammonium chloride (13 mL) and the stirred mixture was allowed to warm to room temperature then extracted twice with ethyl acetate. The combined extracts were washed with 10% aqueous sodium chloride solution and then concentrated to approximately a 20 mL solution which was used directly in the next stage.

### (Mass spectroscopy of the solution was consistent with the formation of (1Z,5S)-5-([(tert-butoxy)carbonyl]amino}-1-[dimethyl(oxo)-lambda-6-sulfanyliumyl]-6,6,6-trifluorohex-1-en-2-olate).

The above ethyl acetate solution (20 mL) was treated with O-benzylhydroxylamine hydrochloride and the mixture heated at 60°C for 2.75h then allowed to cool to room temperature before washing with 10% aqueous sodium chloride solution. This solution was reduced in volume to approximately 10 mL and used directly in the next step.

### (Mass spectroscopy of the solution was consistent with the formation of tert-butyl N-[(2S,5E/Z)-5-[(benzyloxy)imino]-6-chloro-1,1,1-trifluorohexan-2-yl]carbamate).

The above ethyl acetate solution (10 mL) was treated with methanesulfonic acid (1.1 mL, 1.7g, 17.4 mmol) and the mixture heated at 45°C for 1h then allowed to cool to room temperature. This was added to a solution of potassium bicarbonate (2.9g, 29 mmol) in water (10 mL) and stirred at 45°C for 3h. After cooling the phases were separated and the ethyl acetate phase washed with 10% aqueous sodium chloride solution. The ethyl acetate solution was used as such in the next stage.

### (Mass spectroscopy of the solution was consistent with the formation of (3E/Z,6S)-N-(benzyloxy)-6-(trifluoromethyl)piperidin-3-imine).

The above ethyl acetate solution was cooled to -15°C and concentrated sulfuric acid (0.6g, 0.3 mL, 6 mmol) was added. Then sodium triacetoxyborohydride (0.5g, 2.4 mmol) was added portion wise, allowing the temperature to rise from -15°C to -5°C over 1h. Water was added followed by concentrated aqueous ammonia (1 mL). The mixture was extracted with ethyl acetate and the organic extract washed with brine, dried and evaporated affording an oil which was chromatographed on silica eluting with 15-40% ethyl acetate in DCM affording (3R,6S)-N-(benzyloxy)-6-(trifluoromethyl)piperidin-3-amine as an oil (184 mg, 12% over 4 stages).
M/z 275 (M+H)⁺

### c. (2S,5R)-6-(Benzyloxy)-2-(trifluoromethyl)-1,6-diazabicyclo[3.2.1]octan-7-one

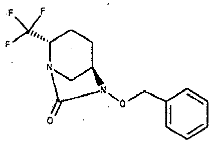

A mixture of (3R,6S)-N-(benzyloxy)-6-(trifluoromethyl)piperidin-3-amine (0.18g, 0.67 mmol) and potassium carbonate (0.53g, 3.82 mmol) in DCM (20 mL) was treated with triphosgene (0.2g, 0.67 mmol) at -10°C. After 30 minutes DMAP (3 mg, 0.03 mmol) was added. The reaction mixture was stirred at room temperature overnight then washed with water, dried (Na2SO4) and evaporated. The residue was chromatographed on silica eluting with an ethyl acetate-DCM gradient affording a colourless solid (0.16g, 81%).
M/z 301.4 (M+H)⁺

### d. Sodium (2S, 5R)-7-oxo-2-(trifluoromethyl)-1,6-diazabicyclo[3.2.1]octan-6-yl sulfate

A solution of (2S,5R)-6-(benzyloxy)-2-(trifluoromethyl)-1,6-diazabicyclo[3.2.1]octan-7-one (163 mg, 0.54 mmol) in iso-propanol (5 mL) was treated with sulfur trioxide trimethylamine complex (85 mg, 0.61 mmol), triethylamine (11 mg, 0.11 mmol), 10% palladium on charcoal (10 mg) and water (0.6 mL). The mixture was hydrogenated under balloon pressure for 4.5h then more sulfur trioxide trimethylamine complex (82 mg, 0.3 mmol) was added. The mixture was stirred under nitrogen for 2h then filtered and concentrated to approximately 1.5 mL. This was diluted with water and treated with saturated aqueous sodium bicarbonate solution (3 mL). The mixture was loaded onto a reverse phase C18 cartridge (lOg size) and eluted with 0-40% ACN in water. Evaporation of product-containing fractions gave a white solid that was redissolved in water and rechromatographed using the same chromatography conditions. Evaporation of product-containing fractions gave a white solid that was redissolved in water and rechromatographed using the similar chromatography conditions except that THF was used in place of ACN. Evaporation gave the title compound as a white solid (86 mg, 51%).
¹H NMR (400 MHz, d6-DMSO) *δ* 4.09-4.06 (1H, m), 3.89-3.78 (1H, m), 3.24 (1H, d, *J* = 12.0 Hz), 3.13-3.08 (1H, m), 1.92-1.74 (4H, m).
¹⁹F NMR (376.4 MHz, d6-DMSO) *δ* -74.7
LCMS (ESI [M-Na]⁻) 289.1, (97.2%).

### Example 2

### Sodium (2S, 5R)-7-oxo-2-(difluoromethyl)-1,6-diazabicyclo[3.2.1]octan-6-yl sulfate

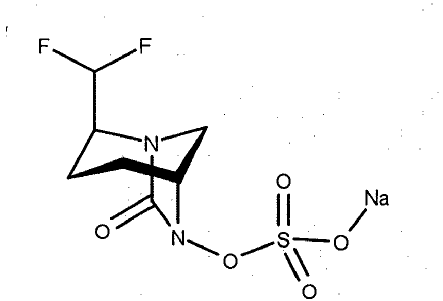

### a. Ethyl (2S,5R)-6-(benzyloxy)-7-oxo-1,6-diazabicyclo[3.2.1]octane-2-carboxylate

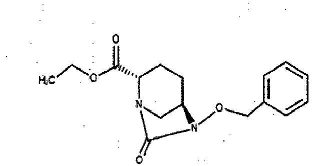

A suspension of ethyl (2S,5R)-5-[(benzyloxy)amino]piperidine-2-carboxylate oxalate salt (15.0g, 40.7 mmol; purchased from Frapps Chemicals Co, Zhejiang, China, http://www.frappschem.com) in THF (150 mL) at 0°C was treated with a solution of potassium hydrogen carbonate (16.3g, 163 mmol) in water (150 mL). After 1 hour the mixture was extracted with ethyl acetate and the combined extracts washed with water then brine, dried (Na2SO4) and evaporated affording ethyl (2S,5R)-5-[(benzyloxy)amino]piperidine-2-carboxylate as a brown oil (10.0g, 88%).

A solution of ethyl (2S,5R)-5-[(benzyloxy)amino]piperidine-2-carboxylate (5.0g, 18 mmol) in DCM (100 mL) at 0°C was treated with DIPEA (12.5 mL, 72 mmol) followed by the addition of a solution of triphosgene (2.6g, 9 mmol) in DCM (10 mL). The mixture was stirred at ambient temperature for 16h then saturated aqueous potassium bicarbonate solution (100 mL) was added. After 1h the phases were separated and the DCM phase washed with water then brine, dried (Na2SO4) and evaporated affording a brown oil (5.0g, 92%) with spectroscopic data consistent with ethyl (2S,5R)-6-(benzyloxy)-7-oxo-1,6-diazabicyclo[3.2.1]octane-2-carboxylate and which was used directly without purification.
M/z 305.4 (M+H)⁺

### b. (2S,5R)-6-(benzyloxy)-2-(hydroxymethyl)-1,6-diazabicyclo[3.2.1]octan-7-one

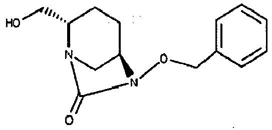

A solution of the crude ethyl (2S,5R)-6-(benzyloxy)-7-oxo-1,6-diazabicyclo[3.2.1]octane-2-carboxylate (5.0g, 16.4 mmol) in THF (75 mL) and ethanol (75 mL) was treated at 0°C with a solution of lithium borohydride in THF (4M; 24 mL, 96 mmol). After 1h at room temperature, the mixture was recooled to at 0°C and a further portion of a solution of lithium borohydride in THF (4M; 12 mL, 48 mmol) was added. After a further 16h at room temperature the mixture was treated with saturated aqueous monopotassium phosphate (200 mL) and the mixture extracted several times with DCM. The combined DCM extracts were washed with water then brine, dried (Na2SO4) and evaporated affording a brown oil (5g). This was chromatographed on silica eluting with 0-100% ethyl acetate in hexane affording a colourless oil (2.2g, 47% over 2 steps).
M/z 263.3 (M+H)⁺

### c. (2S,5R)-6-(Benzyloxy)-7-oxo-1,6-diazabicyclo[3.2.1]octane-2-carbaldehyde

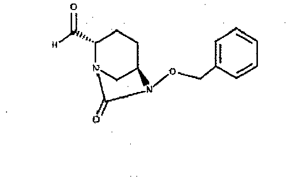

A solution of (2S,5R)-6-(benzyloxy)-2-(hydroxymethyl)-1,6-diazabicyclo[3.2.1]octan-7-one (1.5g, 5.7 mmol) in DCM (50 mL) was treated at 0°C with trichloroisocyanuric acid (1.9g, 8.6 mmol) and TEMPO (90 mg, 0.6 mmol). The mixture was stirred at 0°C for 2h then filtered through celite, washing with DCM. The combined DCM filtrates were were washed with saturated aqueous sodium bicarbonate solution, brine, dried (Na2SO4) and evaporated to afford an oil (1.5g, 100%) which was used directly in the next step.
M/z 261.4 (M+H)⁺

### d. (2S,5R)-6-(Benzyloxy)-2-(difluoromethyl)-1,6-diazabicyclo[3.2.1]octan-7-one

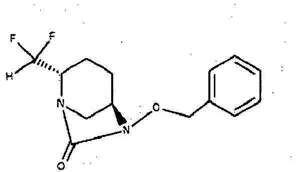

A solution of (2S,5R)-6-(benzyloxy)-7-oxo-1,6-diazabicyclo[3.2.1]octane-2-carbaldehyde (1.5g, 5.7 mmol) in DCM (30 mL) at 0°Cwas treated with DAST (1.5 mL, 12 mmol). The mixture was stirred at room temperature for 4 hours then solvent was removed by purging with nitrogen. The residue was dissolved in ethyl acetate and added to ice-cold water. The organic phase was separated, washed with saturated aqueous sodium bicarbonate solution, brine, dried (Na2SO4) and evaporated affording an oil. This was chromatographed on silica eluting with 20% ethyl acetate in hexane affording a yellow oil (0.7g, 44% over 2 steps).
M/z 283.3 (M+H)⁺

### e. (2S,5R)-2-(Difluoromethyl)-6-hydroxy-1,6-diazabicyclo[3.2.1]octan-7-one

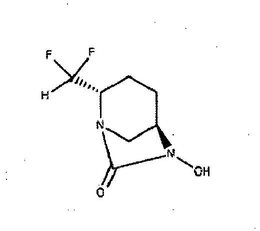

A solution of (2S,5R)-6-(benzyloxy)-2-(difluoromethyl)-1,6-diazabicyclo[3.2.1]octan-7-one (0.60g, 2.1 mmol) in methanol (15 mL) was hydrogenated over 10% palladium on charcoal (0.60g) at 100 p.s.i. in a steel bomb. After 4h the mixture was filtered through celite, washing with methanol and the combined filtrates were evaporated to give a white solid (0.4g, 100%) which was used directly in the next step.
M/z 193.3 (M+H)⁺

### f. Tetrabutylammonium (2S,5R)-2-(difluoromethyl)-7-oxo-1,6-diazabicyclo[3.2.1]octan-6-yl sulfate

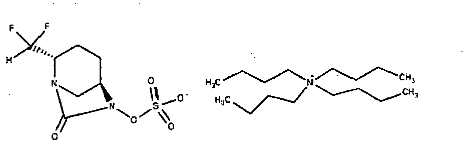

A solution of (2S,5R)-2-(difluoromethyl)-6-hydroxy-1,6-diazabicyclo[3.2.1]octan-7-one (0.40g, 2.1 mmol) in DCM (30 mL) at 0°C was treated with TEA (1.1 mL, 8.3 mmol) then sulfur trioxide pyridine complex (0.67g, 4.2 mmol) was added. After 4h at room temperature a solution of n-tetrabutylammonium acetate (0.94g, 3.1 mmol) in water (20 mL) was added. After 2h further DCM was added and the phases were separated. The DCM phase was washed with water, dried (Na2SO4) and evaporated. The residue was chromatographed on silica eluting with 0-100% ethyl acetate in hexane followed by 4% methanol in DCM, affording a colourless oil (0.45g, 42% over 2 steps).
M/z 271.4 (M⁻)

### g. Sodium (2S, 5R)-7-oxo-2-(difluoromethyl)-1,6-diazabicyclo[3.2.1]octan-6-yl sulfate

A solution of tetrabutylammonnium (2S,5R)-2-(difluoromethyl)-7-oxo-1,6-diazabicyclo[3.2.1]octan-6-yl sulfate (0.45g, 0.88mmol) in water (10 mL) was treated with Dowex™ Na resin (1g). After 1h the mixture was filtered through a bed of Dowex™ Na resin. The combined filtrates were passed through a second bed of Dowex™ Na resin, washing with water (5 mL) then the combined filtrates were freeze-dried to obtain the title compound as a white solid (202 mg, 78%).
¹H NMR (400 MHz, d6-DMSO) *δ* 6.24 (1H, t, J = 4.4 Hz, CHF2), 4.06 (1H, m), 3.40 (1H, m), 3.20 (1H, m), 3.05 (1H, m), 1.95-1.75 (4H, m).
LCMS (ESI [M-Na]⁻) 271.1

### Example 3

### Sodium (2S,5R)-2-(dichloromethyl)-7-oxo-1,6-diazabicyclo[3.2.1]octan-6-yl sulfate

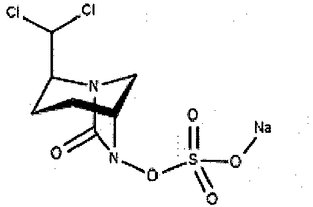

### a. 6-(Benzyloxy)-2-(dichloromethyl)-1,6-diazabicyclo[3.2.1]octan-7-one

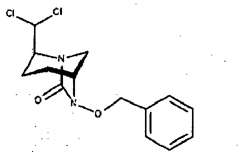

A solution of (2S,5R)-6-(benzyloxy)-7-oxo-1,6-diazabicyclo[3.2.1]octane-2-carbaldehyde (2.2g, 8.4 mmol) in DCM (100 mL) was treated with phosphorus pentachloride (3.5g, 16.9 mmol). After 16h the mixture was diluted with DCM and washed with ice-cold water, brine, dried (Na2SO4) and evaporated. The residue was chromatographed on silica eluting with 0-20% ethyl acetate giving 3 separate fractions. Fractions 1 and 3 did not contain the desired compound but Fraction 2 (TLC; Rf 0.4 in 20% ethyl acetate/hexane), a white solid (130 mg, 5%), showed spectroscopic data consistent with the desired compound. In particular, HMBC NMR studies showed a close spatial proximity between the carbonyl carbon and the C2-H proton, thereby defining the -CCl2H substituent as axial and the C2-chirality as S, as in the diagram.
M/z 315:4, 317.3 (M+H)⁺

### b. 2-(Dichloromethyl)-6-hydroxy-1,6-diazabicyclo[3.2.1] octan-7-one

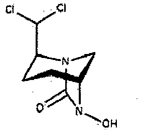

A solution of 6-(benzyloxy)-2-(dichloromethyl)-1,6-diazabicyclo[3.2.1]octan-7-one (130 mg, 0.4 mmol) in methanol (10 ml) was treated with 10% palladium on charcoal (130 mg) and hydrogenated (balloon pressure) for 2h. The mixture was filtered through celite, washing with methanol. The combined filtrates were evaporated to give an off-white solid (85 mg, 92%) which was used immediately in the next stage.
M/z 225.4; 227.3 (M+H)⁺

### c. Tetrabutylammonnium (2S,5R)-2-(dichloromethyl)-7-oxo-1,6-diazabicyclo[3.2.1]octan-6-yl sulfate

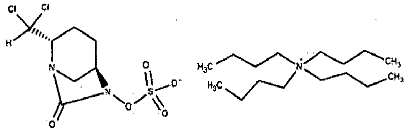

A solution of 2-(dichloromethyl)-6-hydroxy-1,6-diazabicyclo[3.2.1]octan-7-one (85 mg, 0.37 mmol) in DCM (10 mL) was treated at 0°C with TEA (0.2 mL, 0.8 mmol) and sulfur trioxide pyridine complex (0.12g, 0.76 mmol). The mixture was stirred at room temperature for 4h then evaporated to dryness. The residue was redissolved in DMF (2 mL) and treated with TEA (0.4 mL, 1.6 mmol) and pyridine complex (0.12g, 0.76 mmol). The mixture was stirred at room temperature for 2h then a solution of n-tetrabutylammonium acetate (0.28g, 0.8 mmol) in water (10 mL) was added. After 2h, the mixture was diluted with DCM and the organic extract washed with water then brine, dried (Na2SO4) the evaporated to give an oil. This was chromatographed on silica eluting with 0-100% ethyl acetate in hexane then 6% methanol in DCM to afford a colourless oil (68 mg, 33%).
M/z 304.4, 306.3 (M⁻)

### d. Sodium (2S,5R)-2-(dichloromethyl)-7-oxo-1,6-diazabicyclo[3.2.1]octan-6-yl sulfate

A solution of tetrabutylammonnium (2S,5R)-2-(dichloromethyl)-7-oxo-1,6-diazabicyclo[3.2.1]octan-6-yl sulfate (68 mg, 0.12 mmol) in water (2 mL) and ACN (2 mL) was treated with Dowex™ Na resin (0.5g). After 1h the mixture was filtered through a bed of Dowex™ Na resin. The combined filtrates were passed through a second bed of Dowex™ Na resin, washing with water-ACN (1 mL; 1 mL). The filtrates were passed through a second bed of Dowex™ Na resin then the combined filtrates were freeze-dried to obtain the title compound as a white solid (36 mg, 88%).
¹H NMR (400 MHz, d₆-DMSO) *δ* 6.25 (1H, d, J = 0.8 Hz, CHCl2), 4.02 (1H, m), 3.45 (1H, m), 3.22 (1H, d), 2.95 (1H, m), 1.95-1.75 (4H, m).
LCMS (ESI [M-Na]⁻) 303.4, 305.4

### Biological Activity

Experiments were conducted to determine:
(1) The inhibitory activity of the compounds of the invention against Serine β-lactamase enzymes from different classes;
(2) β-lactam potentiation by the compounds against strains expressing SBL enzymes

Details of the protocols used for each of the sets of experiments are set out below:

### Enzymatic inhibition

### In vitro enzyme inhibition assays

Enzyme inhibition assays were performed using purified SBL enzymes (such as TEM-1; AmpC; CTX-M15; KPC-2; OXA-48) in 10mM HEPES buffer pH 7.5 in 96-well microtiter plates. Nitrocefin (100µM for TEM-1, AmpC, KPC-2, OXA-48; and 50µM for CTX-M15) was used as substrate. Its hydrolysis was followed, after an initial 10min incubation at 30°C, at 482 nm during 12mn every 30 seconds using a Perkin Elmer Envision UV fluorescence plate reader. Hydrolysis rate data in presence of a range of inhibitors was analysed and IC₅₀ determined for each compound using Dotmatics database software.

Compound dilution was performed in DMSO.

Mean IC50 results are shown below for enzymatic inhibition of a selected panel of SBL enzymes. Data are banded together in the following manner:-

| | |
|---|---|
| KPC-2: | IC50 < 10 nM (A); > 10 nM (B). |
| AmpC: | IC50 < 10 nM (A); 10 - 40 nM (B); 40 - 60 nM (C). |
| OXA-48: | IC50 < 20 nM (A); 20 -100 nM (B); 100 - 200 nM (C); 200 - 300 nM (D). |
| CTX-M15: | IC50 < 5 nM (A); 5 - 20 nM (B); 20 - 100 nM (C); 100 - 200 nM (D). |

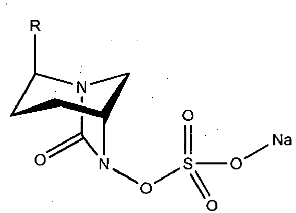

| Compound | *R* substituent | KPC-2 | Amp-C | OXA-48 | CTX-M15 |
|---|---|---|---|---|---|
| Avibactam | CONH2 | 7.8 (A) | 8.0 (A) | 296.6 (D) | 1.4 (A) |
| Example 1 | CF3 | (A) | (A) | (A) | (B) |
| Example 2 | CHF2 | (A) | (B) | (B) | (B) |
| Example 3 | CHCl2 | (A) | (C) | (C) | (C) |

### Antimicrobial susceptibility testing

### Antibiotic activity of a β-lactam antibiotic on SBL/ESBL expressing bacteria in the presence of the compounds of the invention

The experiments were carried out using the 'broth micro-dilution method' according to the protocols M07-A8 established by the Clinical Laboratory Standards Institute (CLSI). Serial dilutions of the β-lactam antibiotic Meropenem were prepared in 96-well plates in cation-adjusted Mueller-Hinton broth (CAMHB); the concentration range was defined from 0.03 mg/L to 512 mg/L. A bacterial inoculum of each strain (clinical isolates) was adjusted to a 0.5 McFarland turbidity standard in physiologic serum (0.9 % NaCl), then diluted 1:100 in CAMHB and added to each well to give a final bacterial cell number of 5x10⁵ CFU/well. After incubation for 18-20 hours in a heating chamber at 37°C, the growth inhibition was evaluated by the absence of any bacterial development.

Minimal inhibitory concentrations (MICs) are taken as the lowest concentration of antibiotic at which the test organism did not show visible growth; results were confirmed by measuring the optical density (OD) at 600 nm in a spectrophotometer.

Compounds of the invention were tested at concentrations of 4 µg/mL. The clinical strains used in these Meropenem potentiation experiments were NTBC091.1 (*E. coli* strain expressing KPC-2, TEM-1); NTBC093 (*E. cloacae* strain expressing KPC-2, TEM-1); NTBC096.1 (*K. pneumoniae* strain expressing OXA-181 and SHV-11); NTBC099(*K. pneumoniae* strain expressing KPC-3, SHV-11 and TEM-1); NTBC189 (*K. pneumoniae* strain expressing TEM-OSBL(b), CTX-M-14, OXA-48(c)).

| Strain number | β-Lactamase enzyme(s) | Classification | MIC mero, µg/mL, no inhibitor | MIC mero + 4 µg/mL Example1 | MIC mero + 4 µg/mL Example2 |
|---|---|---|---|---|---|
| NTBC091.1 | KPC-2 +TEM-1 | E. coli | 4 | 0.03 | 0.03 |
| NTBC093 | KPC-2 +TEM-1 | E. cloacae | 8 | 0.06 | 0.06 |
| NTBC096.1 | OXA-181 +SHV-11 | K. pneumo | 32 | 4 | 4 |
| NTBC099 | KPC-3 + SHV-11 + TEM-1 | K. pneumo | 256 | 2 | 1 |
| NTBC189 | TEM-OSBL(b) + CTX-M + OXA-48(c) | K. pneumo | 32 | 2 | 2 |

## Claims

1. A compound which is a diazabicyclooctanone of Formula (I) or a pharmaceutically acceptable salt thereof: 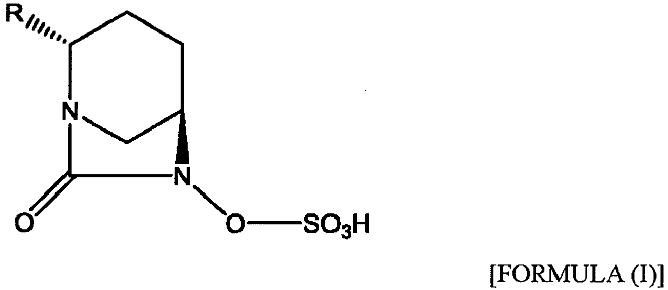 wherein
∘ R is selected from C(O)R¹ and C₁₋₄ alkyl, wherein the C₁₋₄ alkyl group is substituted with at least one halogen atom and is optionally further substituted with one or two substituents R²;
∘ R¹ is C₁₋₄ alkyl which is substituted with at least one halogen atom and is optionally further substituted with one or two substituents R²;
∘ each R² is independently selected from OH; C₁₋₄ alkoxy which is unsubstituted or substituted with one or more halogen atoms; C(O)R³; C(O)OH; C(O)OR³; 6- to 10-membered aryl; 5- to 6-membered heteroaryl; and 4- to 6-membered heterocyclyl; wherein the aryl, heteroaryl and heterocyclyl groups are unsubstituted or substituted with one, two or three substituents selected from halogen, OH, C(O)R³, C(O)OH, C(O)OR³ and C₁₋₄ alkyl and C₁₋₄ alkoxy groups which are themselves unsubstituted or substituted with one or more halogen atoms;
∘ R³ is C₁₋₄ alkyl which is unsubstituted or substituted with one or more halogen atoms.

2. A compound according to claim 1, wherein R is selected from C(O)R¹ and C₁₋₄ alkyl, wherein the C₁₋₄ alkyl group is substituted with at least one halogen atom and is optionally further substituted with one substituent R².

3. A compound according to claim 1, wherein R¹ is C₁₋₄ alkyl which is substituted with at least one halogen atom and is optionally further substituted with one substituent selected from OH and C₁₋₄ alkoxy which is itself unsubstituted or substituted with one or more halogen atoms;
preferably R¹ is C₁₋₂ alkyl, wherein the C₁₋₂ alkyl group is substituted with at least two halogen atoms selected from fluorine and chlorine.

4. A compound according to any one of the preceding claims, wherein each R² is independently selected from OH; C₁₋₂ alkoxy which is itself unsubstituted or substituted with one or more halogen atoms; C(O)OR³, wherein R³ is unsubstituted C₁₋₂ alkyl; and unsubstituted 5- to 6-membered heteroaryl;
preferably each R² is independently selected from OH; OMe; C(O)OMe; and unsubstituted thiazolyl.

5. A compound according to claim 1, wherein:
- R is selected from C(O)R¹ and C₁₋₄ alkyl, wherein the C₁₋₄ alkyl group is substituted with at least one halogen atom and is optionally further substituted with one substituent R²;
- R¹ is C₁₋₄ alkyl which is substituted with at least one halogen atom and is optionally further substituted with one substituent selected from OH and C₁₋₄ alkoxy, the C₁₋₄ alkyl group being unsubstituted or substituted with one or more halogen atoms; and
- R² is selected from OH; C₁₋₂ alkoxy which is itself unsubstituted or substituted with one or more halogen atoms; C(O)OR³, wherein R³ is unsubstituted C₁₋₂ alkyl; and unsubstituted 5- to 6-membered heteroaryl.

6. A compound according to claim 1, wherein:
- R is selected from C(O)R¹ and C₁₋₄ alkyl, preferably C₁₋₂ alkyl, wherein the alkyl group is substituted with at least one halogen atom and is optionally further substituted with one substituent R²;
- R¹ is C₁₋₂ alkyl, wherein the C₁₋₂ alkyl group is substituted with at least two halogen atoms selected from fluorine or chlorine; and
- R² is selected from OH; OMe; C(O)OMe; and unsubstituted thiazolyl.

7. A compound according to any one of the preceding claims, wherein R is C₁₋₂ alkyl, wherein the C₁₋₂ alkyl group is substituted with at least one halogen atom and is optionally further substituted with one substituent R².

8. A compound according to claim 1, wherein R is selected from CF₃, CHF₂, CHCl₂, CCl₃, CH₂F, CF₂CH₃, CF₂CH₂CO₂Me, COCF₃, CF₂-thiazolyl, CF₂CH₂OCH₃, CF₂CH₂CH₂OH, CH(OH)CF₃, CH₂CF₃ and CF₂-oxetanyl, preferably from CF₃, CHF₂ and CHCl₂.

9. A pharmaceutical composition comprising a compound according to any one of the preceding claims and a pharmaceutically acceptable carrier or diluent and optionally further comprising (i) an antibiotic agent and/or (ii) a metallo-β-lactamase inhibitor.

10. A combination of a compound according to any one of claims 1 to 8 and one or more of (i) an antibiotic agent and (ii) a metallo-β-lactamase inhibitor.

11. A compound according to any one of claims 1 to 8 for use in the treatment or prevention of bacterial infection by co-administration with an antibiotic agent and/or a metallo-β-lactamase inhibitor.

12. A composition, combination or compound for use according to any one of claims 9 to 11, wherein the antibiotic agent is a carbapenem antibiotic, preferably the antibiotic agent is meropenem.

13. A compound according to any one of claims 1 to 8, or a composition or combination according to any one of claims 9, 10 or 12 for use in the removal or reduction of antibiotic resistance in Gram-negative bacteria.

14. A compound according to any one of claims 1 to 8, or a composition or combination according to any one of claims 9, 10, 12 or 13 for use in the treatment or prevention of bacterial infection.

15. A compound, composition or combination for use according to any one of claims 11 to 14 wherein the Gram-negative bacteria are selected from Enterobacteriaceae, Pseudomonadaceae and Moraxellaceae, or the bacterial infection is caused by bacteria selected from Enterobacteriaceae, Pseudomonadaceae and Moraxellaceae, preferably
(a) the bacteria selected from Enterobacteriaceae, Pseudomonadaceae and Moraxellaceae are selected from *Klebsiella pneumoniae, Escherichia coli, Enterobacter Cloacae*, *Pseudomonas aeruginosa*, *Burkholderia cepacia* and *Acinetobacter baumannii*; and/or
(b) the bacterial infection is caused by Carbapenem Resistant Enterobacteriaceae.
